# EUROPEAN PATENT APPLICATION

(11) **EP 3 574 949 A1**
(43) Date of publication of application: **04.12.2019**
(21) Application number: 18744451.8
(22) Date of filing: 25.01.2018
(51) Int. Cl.: A61M 37/00

(54) **MICRONEEDLE PATCH APPLYING DEVICE**

(30) Priority: 25.01.2017 JP 2017011034
(71) Applicant: Cosmed Pharmaceutical Co., Ltd., Kyoto-shi, Kyoto 601-8014 (JP)
(72) Inventor: QUAN, Ying-shu, Kyoto-shi Kyoto 601-8014 (JP); KAMIYAMA, Fumio, Kyoto-shi Kyoto 601-8014 (JP)
(74) Representative: Ter Meer Steinmeister & Partner
(86) International application number: PCT/JP2018/002186
(87) International publication number: WO 2018/139504

(57) **Abstract**

To provide a microneedle applicator which can be easily attached with the microneedle patch and is not likely to cause skin infection even when continuously administered to a large number of users.

A microneedle patch application device comprising: a movable cylinder having an opening equipped with a clasp or a screw for attaching the microneedle patch case at one end; a case body connected with the other end of the movable cylinder; a piston disposed movably in a direction to the opening of the movable cylinder in the case body; a slide for sliding the piston in an opposite direction to the opening; a slide lock for locking the sliding of the slide in the opposite direction; a spring for applying an impact to the piston by the sliding in the opposite direction and the locking; a safety shaft for suppressing unlocking of the slide lock; and a trigger interlocked with the safety shaft. Although the microneedle patch case at the front end is pressed against a skin, it is replaced for each time of application.

## Description

### Technical Field

The present invention relates to an applicator to which a microneedle patch is attached together with a patch case and which is capable of inserting the microneedle patch.

### Background Art

As a method of administering a drug to a human body, oral administration and transdermal administration are often used. Although injection is a typical transdermal administration method, it is a procedure which takes time, is painful, and further is likely to cause an infection, so many people do not welcome the procedure. In transdermal administration, skin stratum corneum works as a barrier to drug permeation, so only applying the drug on a skin surface does not necessarily cause enough permeability. In contrast, perforation of the corneum by using a minute needle, i.e. a microneedle can remarkably improve drug permeation efficiency compared to the application method. An article in which a large number of the microneedles are integrated on a substrate is a microneedle array. Furthermore, a product in which sheets such as an adhesive sheet for adhering the microneedle array to a skin or a protective release sheet for protecting and supporting the adhesive sheet when applying the microneedle array to a skin are added to the microneedle array in order to facilitate its use is called a microneedle patch.

The microneedle patch is desired to be stored safely and hygienically, delivered to a user, and packaged for safe and easy use after production until use. For that purpose, it is profitable to store, transport and hold the microneedle patch in a patch case convenient for use. When the microneedle array is administered to the skin, the microneedles cannot be easily inserted into the skin only by pressing the microneedle array with a finger because skin is generally flexible, and therefore an applicator is often used as an insertion aid. At this time, if the microneedle array can be attached to the applicator together with the patch case, the user need not directly touch the microneedle array, and thus this process is preferable from the viewpoints of not only hygiene but also convenience for the user.

That is, the patch case according to the present invention refers to a container for storing, transporting and holding the microneedle patch, and furthermore a container which makes it possible to attach the microneedle patch to the applicator while holding the microneedle patch in the case and administer the held microneedles to a subject.

Several storage containers for storing microneedle patches have already been reported (Patent Documents 1 and 2). However, there are few reports on patch cases for attaching the microneedle patch to an applicator while storing the microneedle patch in the case.

In a cylindrical patch holder (corresponding to the patch case) attachable to the applicator body, the microneedle patch is attached to a central attachment shoulder (stepped portion) of the patch holder (Patent Documents 3 and 4). This patch holder is attached to the applicator, and an impact is applied by the applicator, then a fragile attachment area of the microneedle patch is broken and separated, and the microneedle patch leaves the patch holder and pierces the skin surface. In addition, the patch holder can be disposable. Also, other documents disclose similar patch holders (Patent Documents 5, 6, and 7).

In a microneedle array cartridge (corresponding to the patch case) comprising a material web (substrate) and a container (cover), the microneedle array is attached to the applicator together with the cartridge (Patent Document 8).

A patch holder (corresponding to the patch case) to be inserted from the side face of the applicator has an attachment shoulder and can hold the microneedle patch on the lower face with a low adhesiveness (Patent Document 9).

### Prior Art Documents

### Patent Documents

Patent Document 1: Japanese Patent Application Laid-Open No. 2012-213586
Patent Document 2: Japanese Patent Application Laid-Open No. 2014-079622
Patent Document 3: Japanese Unexamined Patent Application No. 2004-510534 (Japanese Patent No. 4104975)
Patent Document 4: Japanese Unexamined Patent Application No. 2009-529400
Patent Document 5: Japanese Unexamined Patent Application No. 2004-510530 (Japanese Patent No. 4198985)
Patent Document 6: Japanese Unexamined Patent Application No. 2007-509706 (Japanese Patent No. 4682144)
Patent Document 7: Japanese Unexamined Patent Application No. 2008-534152
Patent Document 8: Japanese Unexamined Patent Application No. 2008-543528
Patent Document 9: Japanese Patent Application Laid-Open No. 2014-042788

### Summary of Invention

### Problem to be solved

The inventors of the present invention have succeeded in developing a patch case which can be easily attached to the microneedle applicator, if necessary, easily separate the microneedle patch, and is disposable (Japanese Patent Application No. 2016-074692). The problem to be solved by the present invention is to provide a microneedle applicator which can be easily attached with the microneedle patch and is not likely to cause skin infection even when continuously administered to a large number of users.

### Solution to Problem

In order to solve the above problems, first, the inventors of the present invention made a case capable of holding the microneedle patch using a thermoplastic polymer as a material for the case body of the microneedle patch, and using a material capable of heat-fusing with the case body as a material for the support film of the adhesive tape of the microneedle patch (Japanese Patent Application No. 2016-074692). Second, the inventors have found that the intended purpose can be achieved by providing an applicator which has a structure for facilitating attachment of the patch case to the foremost side of the microneedle applicator and preventing contact of the applicator body with a skin in administration, comprises a movable cylinder, a piston, a slide, a slide lock, and a spring which facilitate continuous administration, and separately comprises a safety shaft for suppressing unlocking of the slide lock, and a trigger interlocked with the safety shaft, and the inventors have completed the present invention.

The present invention is as follows.
[1] A microneedle patch application device comprising:
   a movable cylinder having an opening equipped with a clasp or a screw for attaching the microneedle patch case at one end;
   a case body connected with the other end of the movable cylinder so that the movable cylinder can move;
   a piston disposed movably in a direction to the opening of the movable cylinder in the case body;
   a slide for sliding the piston in an opposite direction to the opening;
   a slide lock for locking the sliding of the slide in the opposite direction;
   a spring for applying an impact to the piston by the sliding in the opposite direction and the locking;
   a safety shaft for suppressing unlocking of the slide lock; and
   a trigger interlocked with the safety shaft;
   wherein the movable cylinder is moved, the slide lock is released by pulling the trigger interlocked with the safety shaft, and the piston is moved toward the opening by the impact spring.
[2] The microneedle patch application device according to [1], wherein the slide lock is released after pressing against the skin, the piston is moved toward the opening by the impact spring, and the end face of the piston comes out of the opening toward to the skin surface.
[3] The microneedle patch application device according to [1] or [2], wherein the patch case containing a thermoplastic polymer as a material and holding the microneedle patch comprising a support film capable of heat-fusing with the thermoplastic polymer is held with the clasp or the screw at the front end.
[4] The microneedle patch application device according to [3], wherein the heat-fusible support film is a laminate film, a back face of the laminate film is made of a thermoplastic resin having a melting point of 200°C or less, and a front face of the laminate film is made of a thermoplastic resin having a melting point of 200°C or more.
[5] A microneedle patch case, attached to the microneedle patch application device according to any one of [1] to [4].
[6] The microneedle patch case, containing the thermoplastic polymer as a material, and composed of the microneedle patch comprising the support film capable of heat-fusing with the thermoplastic polymer and the patch case for holding the microneedle patch, wherein a skin contact face of the patch case has a width of 2 mm or more.
[7] The microneedle patch case according to [6], wherein the distance of the skin contact face from the support film is 2 mm or more.
[8] The microneedle patch case according to any one of [5] to [7], further having a holder for accommodating the microneedle patch case.

### Effects of Invention

Since the microneedle patch application device according to the present invention (referred to as "applicator" in some cases in the present specification) has a mechanism for attaching the microneedle patch case to the front face of the applicator, the applicator body does not contact a skin of a subject, and therefore hygiene can be maintained by exchanging the patch case every time even in a case of continuous use. When attaching or detaching the microneedle patch case, the patch case can be easily attached and detached using a clasp or a screw for attaching the patch case, and also a plurality of patch cases can be easily continuously used. In addition, in the applicator, the piston is locked and unlocked by a slide-slide lock mechanism, but the microneedle patch is inserted in a two-stage configuration in which the insertion is started by a safety shaft-trigger mechanism, and therefore the device takes safety and suppression of malfunction into consideration.

### Brief Description of Drawings

FIG. 1 illustrates an aspect of a microneedle patch application device, showing a front view, a side view, a rear view and a sectional view (A-A) of the device in an order from the left.
FIG. 2 illustrates a perspective view of an aspect of a microneedle patch case to be used in the present invention.
FIG. 3 illustrates a perspective view of the microneedle patch case in FIG. 2 attached to a front face of the applicator.
FIG. 4 illustrates a perspective view of an aspect of the microneedle patch case body, in which a skin contact face of the patch case body has a ring shape, A represents an outer diameter of the skin contact face of the patch case, B represents a height from the skin adhesion face of the patch case, and C represents a width of the skin contact face of the patch case.
FIG. 5 illustrates a perspective view of an aspect of a holder for accommodating the microneedle patch case.
FIG. 6 illustrates a plane view and a side view of the holder in FIG. 5, as well as a drawing of the microneedle patch case attached to the holder.
FIG. 7 illustrates a plane view of an aspect of a combination of a microneedle patch case and a holder.

### Description of Embodiments

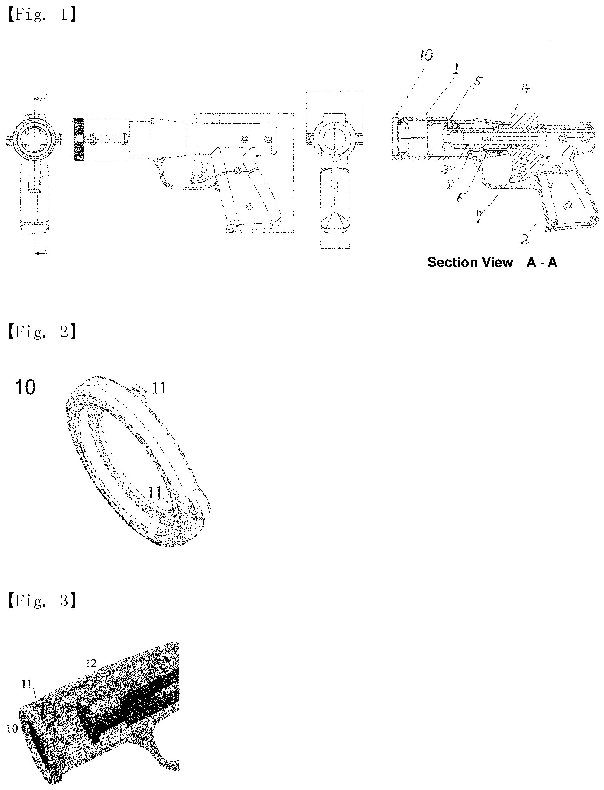

The present invention will be explained with reference to FIG. 1, but the present invention is not limited to the following embodiments.

The applicator is composed of a movable cylinder 1 having an opening equipped with a clasp or a screw for attaching a microneedle patch case 10 at one end, and a case body 2 connected with the other end of the movable cylinder 1 so that the movable cylinder 1 can move. The movable cylinder 1 typically has a hollow cylindrical shape, but may have a hollow prismatic shape. The opening at one end of the movable cylinder 1 is located on the front face of the applicator and is equipped with a clasp or a screw for attaching the microneedle patch case 10. The shape of the clasp or the screw is not particularly limited as long as it can allow the microneedle patch case to be attached and held.

The case body 2 has a shape connectable with the movable cylinder 1, and the shape is not particularly limited as long as it can house a slide, a slide lock, a piston, a spring, a safety shaft, a rotation shaft, and the like. In view of installation of the trigger and operability, the case body of the applicator preferably has a pistol shape.

The materials for the movable cylinder 1 and the case body 2 are not particularly limited.

In the case body 2, a movable piston 3 is disposed movably toward the opening of the movable cylinder 1. The piston 3 allows microneedles to pierce a skin in such a way that the microneedle patch housed in the microneedle patch case 10 is pressed from a back face. This mechanism is as follows.

The piston 3 is moved in an opposite direction to the opening direction by a slide 4 for sliding the piston 3 in the opposite direction to the opening direction, and locked by a slide lock 5 for locking sliding of the slide 4 in the opposite direction. Furthermore, a spring for applying an impact to the piston 3 is provided behind the piston 3, the spring is contracted by sliding the piston 3 in the opposite direction and locking the piston 3, to retain an impact force.

On the other hand, the slide lock 5 is structured such that the unlocking of the lock is started by moving the movable cylinder, specifically pressing the opening of the movable cylinder 1 against the skin. In a case of disengagement of a normal latch mechanism, the locking of the piston is unlocked concurrently with unlocking of the lock, to push the piston, but in the present invention, a safety shaft 6 for suppressing the unlocking, and a trigger 7 interlocked with the safety shaft 6 are further provided. Thus, since the microneedle patch is not inserted immediately after pressing the patch against the skin, an application position on the skin can be accurately determined compared to the conventional applicator using the latch mechanism.

The safety shaft 6 releases suppression of the unlocking through a rotation shaft 8 by pulling the interlocked trigger 7, and the piston moves to the opening by the impact spring to push out the microneedle patch from the back face.

When the piston is moved to the opening to push out the microneedle patch from the back face, it is preferable to adjust the length of the piston, the impact spring, or the like so that the end face of the piston comes out of the opening toward the skin surface. Thereby, the applicator exhibits a strong pressure on the skin and can prevent the microneedles from coming off from the skin.

The microneedle patch case to be attached to the opening of the movable cylinder 1 retains a shape that fits in the clasp or screw provided on the opening. FIG. 2 illustrates an aspect of a microneedle patch case equipped with a clasp. FIG. 3 illustrates a state that the microneedle patch case equipped with the clasp is attached to the front face of the applicator.

Preferably, the microneedle patch case contains a thermoplastic polymer as a material and holds the microneedle patch including a support film capable of heat-fusing with the thermoplastic polymer. The microneedle patch is located on a "lower part" in the patch case i.e. a part close to the skin in use. The heat fusion between the microneedle patch and the patch case does not require a special method, and it is enough to heat the tip of the pointed metal rod to not lower than the melting point of the thermoplastic polymer and press the metal rod tip against the heat-fused part. In addition to the heat fusion at the temperature of not lower than the melting point of the thermoplastic polymer, heat fusion by high frequency heating, heat fusion by laser heating, and the like can also be used.

For facilitating heat fusion, the material of the support film is preferably a thermoplastic polymer film or a non-woven fabric. When the support film is a laminate of a plurality of films (laminated film), if the back face side is a thermoplastic polymer film, the front face side may be any film as long as the front face side can be coated with an adhesive and used as an adhesive sheet to adhesively fix the microneedle array at the center of the sheet.

In the present specification, when the microneedle array is adhesively fixed to the support film, the face with the microneedle array is defined as a front face of the support film, and the opposite face is defined as a back face. In the present invention, the peripheral part of the back face of the support is heat-fused to the patch case.

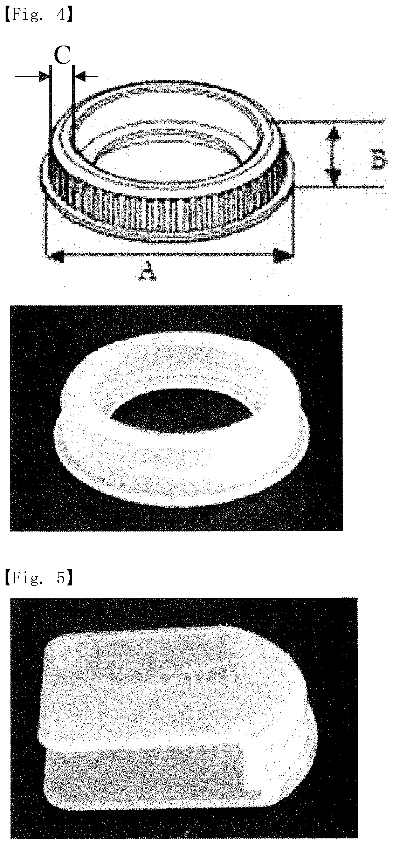

In another aspect of the microneedle patch case, a shape fitted with the screw provided on the opening of the applicator is held at an upper part of the patch case, a lower part has a ring shape as a skin contact face, and the skin contact face has an annular shape surrounded by two concentric circles. The skin contact face can be expressed as a width of the patch case on the skin contact face, and if the width of the skin contact face is narrow, pain may be caused when the patch case is pressed against the skin. The width of the skin contact face is preferably 2 mm or more, more preferably 3 mm to 10 mm. When the thickness of the patch case is increased, the patch case becomes heavy, and therefore it is also preferable that the patch case body is made hollow, or grooves are formed on the side face of the patch case, if necessary. The patch case shown in FIG. 4 has grooves on the side face, A represents an outer diameter of the skin contact face of the patch case, B represents a height from the skin adhesion face of the patch case, and C represents a width of the skin contact face of the microneedle patch case.

The support film to be heat-fused to the microneedle patch case is at a predetermined distance from the skin contact face. The distance of the skin adhesion face from the support film is 2 mm or more, preferably 2 mm to 20 mm. The distance between the support film and the skin adhesion face is appropriately adjusted so that the microneedles can securely pierce the skin.

The patch case body is not particularly limited as long as it is made of a thermoplastic resin which is solid at normal temperature. For the patch case body, a polyolefin-based resin, a polyvinyl chloride, a polycarbonate, a nylon-based resin, a polyethylene terephthalate (PET), or the like can be used, and above all, the polyolefin-based resin is preferable because a resin having a low heat molding temperature can be easily molded. Specifically, a polyethylene, a polypropylene, an ethylene-propylene copolymer, an ethylene-vinyl acetate copolymer, and the like are preferable.

The support film is preferably a thermoplastic resin or a non-woven fabric, but the material of the support film is preferably the same as the material of the patch case because the resins made of the same materials can be easily heat-fused. Specifically, similarly to the patch case, for the support film, i a polyolefin-based resin (polyethylene: melting point of 140°C, polypropylene: melting point of 180°C), a polyvinyl chloride (melting point of 280°C), a nylon-based resin (the melting point significantly varies depending on the composition), a polyethylene terephthalate (PET) (melting point of 270°C), or the like may be used. For the support film and the support non-woven fabric, a single thermoplastic resin may be used, but a laminate film in which a heat-fused face and an adhesive face are made of different resins may be used. When the support film is a laminate film and the patch case is made of a polyolefin, the back face side of the support film is fused to the patch case, and thus the back face is preferably made of a resin having a relatively low melting point (preferably 200°C or less) on the support film e.g. a polyolefin such as polyethylene and polypropylene, and the front face of the support film is preferably made of a thermoplastic polymer having a high melting point (preferably 200°C or more) e.g. a polyethylene terephthalate. In this case, difference in the polarity between the both polymers demonstrates that the polyolefin improves the heat fusion property, and the polyethylene terephthalate is superior to the polyolefin for applying and stably maintaining the adhesive.

The microneedle patch case may further have a holder. The holder accommodates the microneedle patch case and can protect the microneedles during transportation and storage. One or more projections are formed inside the holder. The projections can prevent the microneedle patch case from slipping out from the holder. Particularly, it is more preferable to form the projections on two places at both ends in the direction of taking out the microneedle patch case in the holder. The material of the holder may be the same as the material of the patch case body, and a polyolefin-based resin is desirable because a resin having a low heat molding temperature can be easily molded. Specifically, a polyethylene, a polypropylene, an ethylene-propylene copolymer, an ethylene-vinyl acetate copolymer, and the like are preferable. They are flexible to some extent.

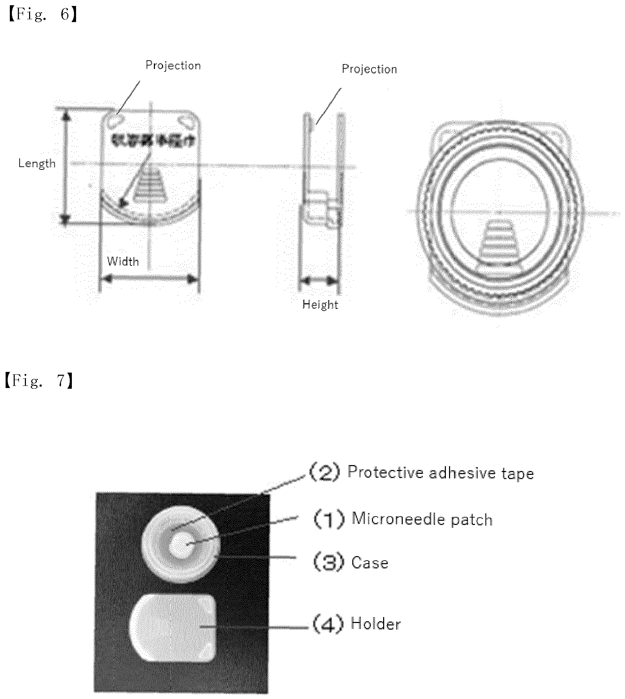

For use, the microneedles are taken out together with the microneedle patch case from the holder, and attached to the microneedle patch application device to administer the microneedles to a skin. FIG. 5 illustrates an aspect of the holder. FIG. 6 illustrates a plane view and a side view of the holder in FIG. 5, as well as an aspect of the microneedle patch case attached to the holder. The microneedle patch case is inserted and ejected in the lengthwise direction of the holder. The width and height of the holder can be appropriately set depending on the size of the microneedle patch case. The projections for fixing the microneedle patch case are formed inside the holder.

FIG. 7 illustrates a plane view of an aspect of a combination of the microneedle patch case and the holder. The microneedle patch (1) is disposed at the center of the microneedle patch case (3) as an upper stage, and the protective adhesive tape (2) for fixing the microneedle patch (1) to the skin is disposed around the microneedle patch (1). The microneedle patch case (3) as the upper stage is inserted and ejected from the left lateral direction by the holder (4) as a lower stage.

When the microneedle patch case is attached to the microneedle patch application device according to the present invention and impacted at a high speed using a piston, the heat-fused part is peeled, and the microneedle patch is administered to the skin together with the support film. In principle, the patch case is disposable. In the present invention, since the patch case is attached to the foremost side of the microneedle patch application device, the applicator does not contact the skin of the subject during administration of the microneedle patch. Furthermore, since the patch case is disposable, there is no risk of skin infection even when the microneedle patches are continuously administered to a large number of subjects.

### Reference Numerals

- 1: Movable cylinder
- 2: Case body
- 3: Piston
- 4: Slide
- 5: Slide lock
- 6: Safety shaft
- 7: Trigger
- 8: Rotation shaft
- 10: Microneedle patch case
- 11: Clasp
- 12: Applicator

## Claims

1. A microneedle patch application device comprising:
a movable cylinder having an opening equipped with a clasp or a screw for attaching the microneedle patch case at one end;
a case body connected with the other end of the movable cylinder so that the movable cylinder can move;
a piston disposed movably in a direction to the opening of the movable cylinder in the case body;
a slide for sliding the piston in an opposite direction to the opening;
a slide lock for locking the sliding of the slide in the opposite direction;
a spring for applying an impact to the piston by the sliding in the opposite direction and the locking;
a safety shaft for suppressing unlocking of the slide lock; and
a trigger interlocked with the safety shaft;
wherein the movable cylinder is moved, the slide lock is released by pulling the trigger interlocked with the safety shaft, and the piston is moved toward the opening by the impact spring.

2. The microneedle patch application device according to claim 1, wherein the slide lock is released after pressing against the skin, the piston is moved toward the opening by the impact spring, and the end face of the piston comes out of the opening toward to the skin surface.

3. The microneedle patch application device according to claim 1 or 2, wherein the patch case containing a thermoplastic polymer as a material and holding the microneedle patch comprising a support film capable of heat-fusing with the thermoplastic polymer is held with the clasp or the screw at the front end.

4. The microneedle patch application device according to claim 3, wherein the heat-fusible support film is a laminate film, a back face of the laminate film is made of a thermoplastic resin having a melting point of 200°C or less, and a front face of the laminate film is made of a thermoplastic resin having a melting point of 200°C or more.

5. A microneedle patch case, attached to the microneedle patch application device according to any one of claims 1 to 4.

6. The microneedle patch case, containing the thermoplastic polymer as a material, and composed of the microneedle patch comprising the support film capable of heat-fusing with the thermoplastic polymer and the patch case for holding the microneedle patch, wherein a skin contact face of the patch case has a width of 2 mm or more.

7. The microneedle patch case according to claim 6, wherein the distance of the skin contact face from the support film is 2 mm or more.

8. The microneedle patch case according to any one of claims 5 to 7, further having a holder for accommodating the microneedle patch case.
